Europäisches Patentamt

European Patent Office

Office européen des brevets

(11) Publication number: 0 292 984
A2

## (12) EUROPEAN PATENT APPLICATION

(21) Application number: 88108482.6

(22) Date of filing: 27.05.88

(51) Int. Cl.⁴ **C12N 15/00 , C12P 21/02 , C07K 13/00 , C12N 1/20 , //(C12N1/20,C12R1:19)**

Claim for the following Contracting State: ES.

The microorganism(s) has (have) been deposited with American Type Culture Collection under number(s)ATCC - 67409 and ATCC - 67410.

(30) Priority: 29.05.87 US 55228

(43) Date of publication of application:
30.11.88 Bulletin 88/48

(84) Designated Contracting States:
AT BE CH DE ES FR GB GR IT LI LU NL SE

(71) Applicant: THE GENERAL HOSPITAL CORPORATION
55 Fruit Street
Boston MA 02114(US)

(72) Inventor: Ronson, Clive
23 Damon Park
Arlington, MA 02174(US)
Inventor: Ausubel, Frederick
271 Lake Avenue
Newton, MA 02161(US)

(74) Representative: Klein, Otto, Dr. et al
Hoechst AG Zentrale Patentabteilung
Postfach 80 03 20
D-6230 Frankfurt am Main 80(DE)

(54) Cloned rhizobium meliloti ntrA (rpoN) gene.

(57) The invention relates to the cloning of an ntrA-like (glnF, rpoN) gene of Rhizobium meliloti. The R. meliloti ntrA product (NtrA) is required for C4-dicarboxylate transport as well as for nitrate assimilation and symbiotic nitrogen fixation. The invention relates as well to the DNA sequence containing the ntrA gene, plasmids containing the gene, transformed hosts containing the plasmids, and to the ntrA gene product.

Figure 1

# CLONED RHIZOBIUM MELILOTI ntrA (rpoN) GENE

## FIELD OF THE INVENTION

The invention belongs to the field of bacterial genetics.

## BACKGROUND OF THE INVENTION

In the enteric bacteria Escherichia coli, Klebsiella pneumoniae, and Salmonella typhimurium, the products of three genes, ntrA (glnF, rpoN), ntrB (glnL) and ntrC (glnG) regulate the transcriptional activation of several operons involved in the assimilation of poor nitrogen sources (22,24). The products of ntrB (NtrB) and ntrC (NtrC) are bifunctional regulatory proteins that act in concert to effect transcriptional repression or activation (22,23,24). The functional state of NtrB is modulated by the products of the glnB and glnD genes in response to the intracellular glutamine to 2-ketoglutarate ratio (3). Depending on its state, NtrB is able to switch NtrC between repressor and activator forms by phosphorylating or dephosphorylating it (31). NtrB in its C-terminal domain and NtrC in its N-terminal domain share homology with a variety of two-component bacterial regulatory systems that may therefore share the same mode of signal transduction (11,32).

The requirement for the ntrA product (NtrA) for transcriptional activation by NtrC has recently been explained by the demonstration that NtrA is a sigma factor required to confer specificity on core RNA polymerase for NtrA-specific promoter sequences (17,19). Promoters activated by NtrC + NtrA do not contain canonical -35 and -10 sequences but instead have the consensus -26 CTGGYAYR-$N_4$-TTGCA-10 (16). NtrA is able to bind to core RNA polymerase, and transcription from the Ntrc-regulated promoter glnAp2 can be activated in vitro by highly purified core enzyme together with NtrA and NtrC (17,19). Based on these observations, it has been proposed that ntrA (glnF) be renamed rpoN (19).

The regulation of the ntrA gene has been studied in K. pneumoniae (7,28) and E. coli (5). In each case, constitutive expression of the ntrA promoter independent of nitrogen source was observed, suggesting that the cells always contain a sub-population of core RNA polymerase bound with NtrA. The ntrA gene from K. pneumoniae has been sequenced and a potential constitutive promoter was identified (27) on the basis of homology to the canonical E. coli promoter (-35 TTGACA-$N_{17}$-TATAAT-10).

In K. pneumoniae, the nitrogen fixation regulatory operon nifLA is one of the operons activated by NtrC + NtrA (reviewed in ref. 16). Like NtrC, the nifA product (NifA) is a transcriptional activator which requires the concerted action of NtrA for the expression of the other six nif operons (16). NifA and NtrC share strong homology in their central and C-terminal domains (4,11) and NifA-activated promoters have a similar consensus to Ntrc-activated promoters (16).

Homologues of the enteric ntrC and nifA genes have been identified in the symbiotic diazotroph Rhizobium meliloti (44,45), and homologues of nifA have been identified in several other Rhizobium and Bradyrhizobium species as well (see ref. 16 for a review). In R. meliloti, NtrC is required for the utilization of nitrate as nitrogen source and the activation of the nifH gene in free-living culture in response to nitrogen limitation (44), while NifA is required for the activation of nif genes, including nifH, in the symbiotic state (45). In contrast to K. pneumoniae, R. meliloti NtrC is not required for the activation of the nifA gene (44). The R. meliloti NtrC and NifA proteins (4,44) and the promoters that they activate (2,42) show strong homology to their enteric counterparts, suggesting that R. meliloti also contains an ntrA gene. However, such a gene has not been identified, despite extensive searches based on hybridization to the K. pneumoniae ntrA gene and complementation of E. coli ntrA mutants. Recently, homologues of ntrC and nifA have also been found in Rhizobium strain ORS571, the stem nodulating symbiont of Sesbania rostrata.

## SUMMARY OF THE INVENTION

In a recent study of the genetic regulation of C4-dicarboxylate transport in Rhizobium leguminosarum dctA a structural gene required for the transport of succinate, fumarate and malate, was found to contain an NtrA-like promoter sequence. Two positive regulatory genes, dctB and dctD, required for activation of dctA transcription were also identified. Interestingly, the C-terminus of dctB gene product (DctB) is homologous to the C-terminus of NtrB and the N-terminus of dctD product (DctD) is homologous to the N-terminus of NtrC. Further, DctD also shares strong homology with NtrC and NifA in the central domain of these proteins that is postulated to interact with NtrA. These observations suggest that NtrA might be required for the expression of dctA: therefore, a selection scheme was formulated for mutations in a putative R. meliloti ntrA gene based on the supposition that NtrA is required for dctA expression.

The invention relates to R. meliloti ntrA mutants, the cloning of the R. meliloti gene, and hosts containing said gene. The invention also relates to the DNA sequence of the R. meliloti ntrA gene and to the amino acid sequence of the ntrA gene product.

The invention relates as well to a method of obtaining a bacterial cell having a mutation in the ntrA gene, which includes:

a) transforming a culture of bacterial cells with a plasmid having an ntrA regulatable gene fusion, the gene fusion resulting from the linkage of an ntrA regulated promoter with a gene whose product has detectable activity,

b) screening said transformed cells for said bacterial cell having a mutation in the ntrA gene, said cell being identified by the absence of said detectable activity, and

c) separating said bacterial cell from the other cells of said culture.


## DESCRIPTION OF THE FIGURE


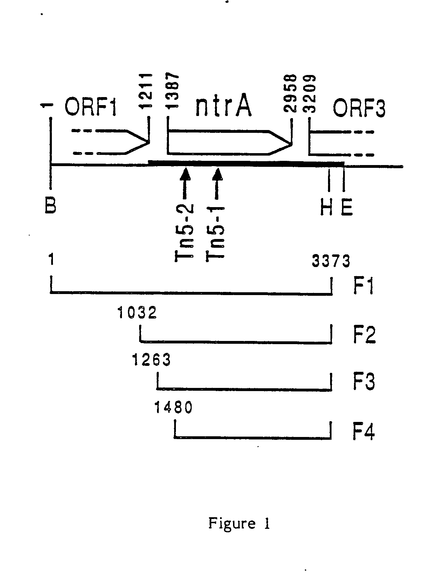

Figure 1. Genetic organization of the 3.5 kb BamHI/EcoRI fragment containing ntrA, and a map of fragments used for complementation tests. The numerals refer to nucleotides from the BamHI site and correspond to those in Fig. 2. The precise start site of ORF1 is unknown but is between 300 and 500 bp from the BamHI site (unpublished data). The sites of the Tn5 insertions in strains Rm1680 (ntrA1::Tn5) and Rm1681 (ntrA2::Tn5) are marked. The heavy line indicates the region whose sequence is presented in Fig. 2. B, BamHI; E, EcoRI; H, HindIII.


## Description of the Preferred Embodiments


The present invention relates to the cloned ntrA gene and its expression in a transformed host. A DNA fragment comprising the ntrA gene is isolated from R. meliloti and inserted into a cloning vector. Another aspect of the invention relates to transformed hosts containing the ntrA gene.

In one embodiment, the invention comprises a bacterial cell which, in its untransformed state, does not produce NtrA but, when transformed according to the invention, can produce ntrA. A recombinant DNA molecule coding for NtrA can be used to transform a bacteria cell using any of the techniques commonly known to those of ordinary skill in the art. Methods for preparing fused, operably linked genes and expressing them in bacteria are known and are shown, for example, in the United States Patent No. 4,366,246. The genetic constructs and methods described therein can be utilized for expression of ntrA in prokaryotic hosts.

Prokaryotic hosts may include Gram negative and Gram positive bacteria, such as E. coli, S. typhimurium, Serratia marcescens and Bacillus subtilis. In general, expression vectors containing promotor sequences which facilitate the efficient transcription of the inserted gene fragment, which are derived from species compatible with the host cells, are used in connection with the prokaryotic host.

The expression vector typically contains an origin of replication, promotor(s), terminator(s) as well as specific genes which are capable of providing phenotypic selection in transformed cells. The transformed host can be fermented and cultured according to means known in the art to achieve optimal cell growth. Examples of prokaryotic promotors which can be used in the invention are rec A, trp, lac, tac, and bacteriophage $p_R$ or $p_L$. Examples of plasmids which can be used in the invention are listed in Maniatis et al., Molecular Cloning, Cold Spring Harbor Laboratory, 1982.

The invention is related to the discovery that R. meliloti contains an ntrA-like gene whose product is required for at least three physiological processes: 1) growth on potassium nitrate as sole nitrogen source, 2) the activation of nif operons in the symbiotic state, and 3) the activation of the C4-dicarboxylate transport gene dctA in response to C4-dicarboxylates. In each case, R. meliloti NtrA acts in conjunction with another transcriptional activator: NtrC, NifA and DCtD respectively. All three of these activators share strong homology in their central regions (4,44), suggesting that this region is involved in the interaction with ntrA product. In addition, the operons they activate contain the NtrA-consensus promoter sequence, consistent with the finding that NtrA is a sigma factor (17,19). These observations suggest that the presence of the NtrA-consensus promoter sequence is diagnostic of an operon that requires NtrA for activation, and that the presence of the conserved central domain in a regulatory protein is predictive of a system that requires NtrA as a co-activator.

The ntrA gene was initially identified on the basis of its role in the activation of nitrogen-regulated operons (15). However, only DctA and not DctD is required for symbiotic nitrogen fixation, suggesting that dctD has not evolved specifically to take advantage of the availability of NtrA in the nodule. Instead, it seems likely that ntrA has a broader physiological role than originally envisaged. Consistent with this idea, NtrA-like promoter sequences have been observed in the xylA (20), carboxypeptidase G2 (30) and pilin (21) genes from Pseudomonas species, and ntrA is required for the xylR-mediated activation of xylA in E. coli - (10). Furthermore, the conserved portion of nifA from K. pneumoniae hybridizes to multiple restriction fragments of Rhizobium strain ORS571 and to multiple restriction fragments of Bradyrhizobium japonicum, R. meliloti and E. coli DNA (T. Adams, Ph.D. Thesis, Michigan State University, East Lansing, 1986). Finally, six strongly hybridizing bands and four weakly hybridizing bands in Bradyrhizobium sp. (Parasponia) DNA probed with a 17 bp oligonucleotide designed from a conserved portion of the central region of nifA, ntrC and dctD genes were observed. Taken together, these results suggest that there may be several additional regulatory genes that require NtrA as co-activator.

It is not clear why genes such as ntrC, nifA and dctD have evolved to use NtrA rather than the rpoD product sigma $^{70}$ for transcriptional initiation; however, the metabolic pathways that these genes regulate are not essential for growth or viability. NtrA mutants of R. meliloti grow as well as wild type in defined medium with glucose as carbon source and ammonium chloride as nitrogen source.

Comparisons of nucleotide sequences surrounding the ntrA gene in R. meliloti (Fig. 2) and K. pneumoniae (27) suggest that genes transcribed in the same direction and possibly in the same operon as ntrA are present upstream (ORF1) and downstream (ORF3) of the ntrA gene in both species (Figs. 1 and 2). However, DNA fragments containing only the R. meliloti ntrA gene were able to complement the R. meliloti ntrA mutants, suggesting that the ntrA promoter lies within 125 bp of the initiation codon of the ntrA gene. Nevertheless, the data does not exclude the possibility that transcription through ORF1 reads through ntrA. and the strong conservation in sequence and location of ORF1 between R. meliloti and K. pneumoniae suggests that ORF1 plays a role in ntrA product the ntrA promoter reads through ORF3.

## Uses

The ntrA gene product is involved in the regulation of genes involved in C4-dicarboxylate transport, nitrate assimilation, and symbiotic nitrogen fixation. The ntrA gene product affects the ability of a microbe to respond to environmental factors. Thus, the invention provides a gene and gene product which involves these functions.

The ntrA gene and gene product is also useful for identifying genes that are involved in pathogenesis. Since bacteria communicate with the host through the ntrA gene product, mutated organisms which do not produce a functional gene product are incapable of receiving the appropriate environmental signals from the host, and the essential pathogenic genes are not activated. Thus, the use of a mutated ntrA gene leads to the identification of pathogenic genes.

## Deposits

Plasmids pUC13NtrA3.5BE and pNtrA10 were deposited in E. coli K12 MC1061 prior to the U.S. filing date at the American Type Culture Collection, Rockville, Maryland, under the auspices of the Budapest Treaty. The accession numbers are as follows:

| Deposited E. coli | Accession Number |
|---|---|
| E. coli K-12 MC1061/pUC13NtrA3.5BE | 67410 |
| E. coli K-12 MC1061/pNtrA10 | 67409 |

## EXAMPLES

**Bacterial Strains, Plasmids and Media.**

The strains and plasmids used are described in Table 1. Construction of the gene bank of Rm1021 DNA in the cosmid vector pLAFR1 has been described (14). Complex media were LB for E. coli and LB or TY for R. meliloti. Defined medium contained RDM salts and vitamins (39), 0.4% glucose as carbon source unless otherwise specified, and 5 mM ammonium chloride as nitrogen source unless otherwise specified. When 10 mM sodium succinate was used as carbon source, the medium was buffered with 50 mM MES, pH 6.1 (38).

Table 1. Bacterial strains and plasmids.

| Strains | Relevant Characteristics | Source or Reference |
|---|---|---|
| **Escherichia coli** | | |
| HB101 | ara xyl mtl pro leu thi lacY supE rpsL endA recA hsdR hsdM | H. Boyer |
| JM101 | Delta (lac-pro) supE thi (F' traD36 proAB$^+$ lacI$^q$ lacZ delta M15) | J. Messing |
| MC1061 | ara leu lac gal hsdR rpsL | M. Casadaban |
| MM294 | pro thr endA hsdR supE | M. Meselson |
| **Rhizobium meliloti** | | |
| Rm1021 | wild-type Str$^r$ | 26 |
| Rm1354 | nifA::Tn5 Str$^r$ Nm$^r$ | 45 |
| Rm5002 | ntrC::Tn5 Str$^r$ Nm$^r$ | 44 |
| Rm1680 | ntrA1::Tn5 Str$^r$ Nm$^r$ | this work |
| Rm1681 | ntrA2::Tn5 Str$^r$ Nm$^r$ | this work |
| Rm1682 | ntrA5::Tn5 Str$^r$ Nm$^r$ | this work |
| **Rhizobium leguminosarum** | | |
| R1538 | dctD Str$^r$ | C. Ronson |

6

## Table 1 continued

### Plasmids

| | | |
|---|---|---|
| pRK290 | IncP Tc$^r$ | 9 |
| pWB5A | Derivative of pRK290 with poly-linker containing EcoRI, ClaI, HindIII, XbaI, BglII, PstI, and BamHI sites | W. Buikema |
| pLAFR1 | Derivative of pRK290 with lambda cos site | 14 |
| pSUP106 | IncQ Tc$^r$ Cm$^r$ | 35 |
| pCR63 | pLAFR1 with (dctA-lacZ) dctB$^+$ dctD$^+$ insert | C. Ronson |
| pRKP9a | pRK290 with nifH-lacZ fusion | 44 |
| pRK2013 | rep (ColEl) Mob$^+$ Tra$^+$ Km$^r$ | 9 |
| pRK602 | pRK2013 npt::Tn9 omega::Tn5 | T. Finan & E. Signer |
| pUC8 | Ap$^r$ | 46 |
| pUC13Ntr3.5BE | pUC8 with 3.5 kb BamHI/EcoRI insert containing ntrA | this work |
| pNtrA10 | pLAFR1 cosmid clone containing ntrA | this work |
| pNtrTn1 | pUC8 with 23 kb ntrA1::Tn5 EcoRI fragment from Rm1680 | this work |
| pNtrTn4 | pUC8 with 23 kb ntrA2::Tn5 EcoRI fragment from Rm1681 | this work |
| pNP45 omega | Ap$^r$ Sp$^r$; source of omega fragment | 13,34 |
| M13mp18 | | 47 |

Strain Rm1021 was mutagenized with transposon Tn5 by mating Rm1021 with strain MM294/pRK602 followed by selection for streptomycin, neomycin resistant transconjugants on LB medium. Approximately 10,000 neomycin resistant Rm1021 transconjugants were pooled by resuspending the colonies in LB; the pooled colonies were stored in 20% glycerol at -70°C.

**Bacterial Crosses.**

All crosses were triparental spot matings using MM294 donor strains and the helper strain MM294/pRK2013 (9,37), and streptomycin was used to counterselect the E. coli strains. For identification of complementing cosmids from the gene bank, the mating mixture was streaked for single colonies directly on defined medium containing succinate, tetracycline and streptomycin. For complementation studies using pWB5A derivatives, the R. meliloti transconjugants were first selected on LB medium containing streptomycin and tetracycline, and then screened on appropriate defined media for phenotypic characterization. For complementation studies using pSUP106 derivatives, the mating mixture was streaked directly on defined medium containing succinate, potassium nitrate and streptomycin, without drugs to select for plasmid transfer since the chloramphenicol-resistance gene of pSUP106 is expressed poorly in R. meliloti.

### Growth and beta-Galactosidase Assays.

Strains were grown to late log phase in TY medium, spun down, resuspended in an equal volume of RDM salts and inoculated 1 in 50 into defined medium containing either no nitrogen source (control culture) or the specified nitrogen source. Growth was monitored by measuring the absorbance at 600 nm of the cultures and by plating for single colonies on TY. To assay induction of the nifH::lacZ fusion, cultures were grown in defined medium containing glutamine at either 0.005% (nitrogen limited) or 0.2% (nitrogen excess). Samples were taken at 20 h (mid-log phase) and 44 h (stationary phase) and assayed for beta-galactosidase activity as described (29). Similar values were obtained at each time point. Nodules were assayed for beta-galactosidase activity as described (44).

### Plant Assays.

Plant assays and isolation of bacteria from nodules were done as described (44).

### DNA Manipulations.

Large and small scale plasmid preparations were made by the boiling method (18). Methods for the isolation of genomic DNA (41), labelling of probes by random primer extensions (12), and Southern hybridizations (32) have been described. Standard procedures (25) Were used for restriction enzyme digestions, ligations, gel electrophoresis and transformations.

### DNA Sequencing.

To prepare fragments for sequencing, 20 ug of pNtr3.5BE DNA was linearized by digestion with either EcoRI or BamHI, and then digested in a final volume of 500 ul with Bal31 nuclease at a concentration (usually a 1 in 200 dilution of enzyme obtained from NE Biolabs) predetermined to remove about 200 bp per minute from each terminus. 50 ul aliquots were taken at one minute intervals and made 20 mM with EGTA, ethanol-precipitated, resuspended and then digested with either BamHI or EcoRI respectively. The Bal31 digested fragments were resolved on a 1% low-melting agarose gel, extracted from the agarose, and ligated into M13mp18 cut with SmaI and either BamHI or EcoRI. The ligation mix was transformed into strain MC1061 and plated on a lawn of JM101, and the resultant plaques were used to prepare template DNAs. Sequencing was by the dideoxy method, using a commercial 17-mer as primer and $^{35}$S-dATP as label (1). This procedure yielded about 1.5 kb of sequence from the EcoRI and BamHI ends. To sequence the remainder of the 3.5 kb BamHI/EcoRI fragment, the replicative forms of the M13 phage containing the largest deletion in each direction were purified, and the Bal31 procedure repeated (33). The final sequence was completely double-stranded.

To determine the sequence from the site of the Tn5 insertion in Rm1681, genomic DNA from Rm1681 was digested with EcoRI, ligated to EcoRI-cleaved pUC8, and transformed into MC1061 with selection for ampicillin plus kanamycin resistance. The resultant plasmid containing Tn5 and flanking genomic DNA was cleaved with BamHI or EcoRI plus BamHI, and the fragments containing the arms of Tn5 and flanking Rhizobium DNA were cloned into M13mp18 in the appropriate orientation for sequence determination from the ends of Tn5 using a Tn5-specific primer (40). The Tn5-specified primer 3´ GTTCATCGCAGGACTTG 5´ was synthesized in the lab of J. Smith, Department of Molecular Biology, Massachusetts General Hospital.

## Computer Analysis.

DNA sequences were compiled using the DBAUTO and DBUTIL programs (42), and analyzed using programs from the University of Wisconsin (8). Pair-wise comparisons between proteins were made with the National Biomedical Research Foundation ALIGN or RELATE programs (6), using the mutation data matrix and a gap penalty of 16. The SD scores given represent the number of standard deviations separating the maximum score of the real sequences from the mean of the maximum scores of 100 random permutations of the two sequences (6).

## Selection of R. meliloti ntrA Mutants.

A selective scheme to isolate R. meliloti ntrA mutants was designed based on the hypothesis that ntrA mutants would be unable to activate dctA expression. To monitor dctA expression in R. meliloti, we utilized an in-frame dctA-lacZ fusion present on pCR63, a broad host range plasmid that contains the entire dct regulon from R. leguminosarum. The R. leguminosarum dctA-lacZ fusion was activated to approximately the same extent in response to succinate in both R. leguminosarum and R. meliloti. Furthermore, R. meliloti strain 1021 containing pCR63 formed white colonies on defined medium containing 5-bromo-4-chloro-3-indolyl beta-D-galactopyranoside (X-gal) with glucose as sole carbon source, and blue colonies on defined medium containing X-gal with glucose plus succinate as carbon sources.

Plasmid pCR63 was conjugated from E. coli strain MM294 into a culture of Rm1021 which had previously been mutagenized with transposon Tn5 (see above) and transconjugants were plated on defined medium containing X-gal, glucose, succinate, streptomycin and neomycin. Since the dct regulatory genes dctB and dctD are present on pCR63, it was likely that any mutant of Rm1021 that contained pCR63 and formed white colonies when plated on medium containing X-gal, glucose and succinate, should be mutated in the R. meliloti ntrA gene. Three white colonies were observed amongst approximately 5000 colonies screened; these were purified on the same medium.

Before further physiological studies were done, the three mutant strains were cured of pCR63. Because plasmids which are stable in free-living cells are frequently lost from strains which are recovered from root nodules, the mutant strains carrying pCR63 were inoculated onto alfalfa plants and bacteria isolated from the resultant nodules were screened for tetracycline sensitive colonies. Tetraycycline sensitive derivatives were obtained at a frequency of 40% and one tetracycline sensitive derivative of each of the three mutants, Rm1680 (ntrA1::Tn5), RM1681 (ntrA2::Tn5) and Rm1682 (ntrA5::Tn5), were saved. In subsequent studies, the three mutants were found to display identical phenotypes, and hence in some cases results from only one, RM1681 (ntrA2::Tn5), are reported.

## Phenotypic Properties of Putative ntrA Mutant and Revertant Strains.

Because K. pneumoniae NtrA works in concert with NtrC and NifA to activate genes involved in nitrogen assimilation and nitrogen fixation respectively, the presumptive ntrA mutants Rm1680, Rm1681 and Rm1682 were assayed for NtrC⁻ and NifA⁻ phenotypes. The mutants for DCtD⁻ phenotypes were also assayed (Table 2).

Table 2. Phenotypic properties of R. meliloti dctD, ntrC, nifA and ntrA mutant strains.

| Phenotype | Strains | | | |
|---|---|---|---|---|
| | Rm5002 (ntrC) | Rm1354 (nifA) | R1538 (dctD) | Rm1681 (ntrA2) |
| Growth on succinate | + | + | - | - |
| Induce dctA fusion in response to succinate[a] | + (280)[b] | + (260) | - (7) | - (9) |
| Growth on 0.5mM KNO₃ as as sole nitrogen source | - | + | + | - |
| Induce nifH fusion in nitrogen-limited free-living culture[a] | - (8) | + (210) | n.t.[c] | - (10) |
| Induce nifH fusion in nodules | + (1490) | - (18) | n.t. | - (12) |
| Form Fix+ nodules | + | - | + | - |

[a]Induction assays were carried out as described in Materials and Methods. The dctA-lacZ fusion was carried on pCR63 and the nifH-lacZ fusion on pRKP9a.

[b]Numbers in brackets denote units of beta-galatosidase calculated according to Miller (29).

[c]n.t. not tested

As expected from the results obtained on succinate/glucose X-gal plates, the dctA-lacZ fusion carried on plasmid pCR63 was not activated in strain Rm1681 when the strain was grown on glucose succinate medium. Furthermore the strain was unable to grow on succinate as sole carbon source (Table 2).

R. meliloti ntrC::Tn5 mutants are unable to grow on 0.5 mM potassium nitrate as nitrogen source (44). When Rm1681 (ntrA2::Tn5) and Rm5002 (ntrC::Tn5) were cultured with potassium nitrate as sole nitrogen source, RM1681 was completely unable to grow on concentrations from 0.5 mM to 5.0 mM, whereas Rm5002 was unable to grow on 0.5 mM KNO₃ but grew slowly on 2 mM and 5 mM KNO₃.

R. meliloti ntrC::Tn5 mutants elicit Fix+ nodules which reduce acetylene at wild-type levels whereas nifA::Tn5 mutants elicit Fix- nodules (44,45). Similarly, a nifH-lacZ fusion is activated to wild-type levels in alfalfa root nodules in an ntrC::Tn5 host but is not activated at all in a nifA::Tn5 host (44). In contrast, a nifH-lacZ fusion is not activated during free-living growth on limiting nitrogen in an ntrC::Tn5 host but is partially activated in a nifA::Tn5 host (44). As shown in Table 2, R. meliloti strain Rm1681 (ntrA2::Tn5) elicited Fix⁻ alfalfa root nodules and a nifH-lacZ fusion in Rm1681 was not activated in root nodules or during free-living nitrogen-limited growth. The above results indicate that Rm1681 has the phenotypic properties of Rhizobium ntrC, nifA and dctD mutants, as would be expected for an ntrA mutant.

Because E. coli, K. pneumoniae and S. typhimurium ntrC and ntrA mutants are characterized by glutamine auxotrophy and inability to grow on several amino acids as sole nitrogen source, the ability of R. meliloti strain Rm1681 (ntrA2::Tn5) to grow in defined liquid medium containing glucose as carbon source and either glutamine, histidine, aspartate, arginine, proline, alanine or ammonium chloride at 50 ug/ml as nitrogen source was tested. Both Rm1681 and Rm5002 (ntrC::Tn5) grew at a similar rate to wild type and reached similar final cell densities to wild type in all of these media.

Three revertants of RM1681 (ntrA2::Tn5) able succinate were isolated after plating a culture on defined medium containing succinate as carbon source and ammonium chloride as nitrogen source. Two of the revertants were able to utilize nitrate and were neomycin-sensitive, suggesting that the succinate-negative and nitrate-negative phenotypes of Rm1681 were both caused by the Tn5 insertion. The third revertant was unable to utilize nitrate and was neomycin-resistant, suggesting it was a Dct-specific second-site revertant.

## Molecular Characterization of the Putative ntrA Mutants.

When ³²P-labelled pUC8::Tn5 DNA was used to probe genomic DNA from strains Rm1680 (ntrA1::Tn5), Rm1681 (ntrA2::Tn5), or RM1682 (ntrA5::Tn5) that was digested with EcoRI, a single band of about 23 kb was detected in each case. When the genomic DNA was digested with EcoRI plus BamHI, two bands of 5.0 kb and 4.2 kb were detected in Rm1680 DNA, while two bands of 4.9 kb and 4.3 kb were detected in RM1681 and Rm1682 DNA. Since Tn5 is 5.7 kb long and contains a single BamHI site, these data indicate that each mutant contains a single Tn5 insertion located within an 18 kb EcoRI fragment and a 3.5 kb BamHI/EcoRI or BamHI fragment, and suggests that strains Rm1681 (ntrA2::Tn5) and Rm1682 (ntrA5::Tn5) are isogenic.

Total DNA from Rm1680 (ntrA1::Tn5) and Rm1681 (ntrA2::Tn5) was digested with EcoRI, ligated to EcoRI-cut pUC8 DNA, and transformed into E. coli strain MC1061. Plasmid DNA was isolated from several of the resulting ampicillin + kanamycin resistant transformants; most of these contained pUC8 with the 23 kb EcoRI insert, and representative plasmids derived from strains Rm1680 (pNtrTn1) and Rm1681 (pNtrTn4) were chosen for further study. Restriction enzyme mapping of pNtrTn1 and pNtrTn4 showed that Tn5 was inserted within a 3.5 kb BamHI/EcoRI fragment in each case (see below).

To determine whether Tn5 in strain Rm1681 (ntrA2::Tn5) was inserted into a gene homologous to K. pneumoniae ntrA, the 4.3 kb BamHI and 4.9 kb EcoRI/BamHI fragments from pNtrTn4 that contained Tn5 and flanking genomic sequences were cloned in M13mp18. An oligonucleotide primer homologous to the ends of Tn5 was used to determine the DNA sequence directly adjacent to the insertion site of Tn5. Significant homology at the amino acid level was detected to the N-terminal region of the ntrA gene from K. pneumoniae (see below), confirming that the Tn5 in Rm1681 was inserted into an ntrA-like gene.

## Molecular Cloning of the R. meliloti ntrA Gene.

To clone the R. meliloti ntrA-like gene, a pLAFR1 gene library of Rm1021 DNA was mated into Rm1680, Rm1681 and Rm1682, and four Dct⁺ trans-conjugants from each strain were isolated on defined medium containing succinate as sole carbon source. These Dct⁺ colonies also grew on nitrate as sole nitrogen source. The complementing pLAFR1 cosmids from the twelve Dct⁺ colonies were conjugated into E. coli strain HB101 in a tri-parental mating using MM294/pRK2013 as helper. Plasmid DNA was isolated from the twelve E. coli strains containing the pLAFR1 cosmids. All twelve plasmid DNAs gave identical EcoRI restriction patterns and contained an 18 kb EcoRI fragment.

The 4.3 kb BamHI fragment from pNtrTn4 (see preceding section) that contained part of Tn5 and flanking genomic sequences from Rm1681 was gel-purified, labelled with ³²P, and used to probe the EcoRI digested DNAs from the 12 complementing cosmids using the Southern blotting and hybridization procedure. In each case, hybridization was detected to the 18 kb EcoRI fragment. These results confirm that the mutant phenotypes of Rm1681 were caused by the Tn5 insertion and not an endogenous insertion sequence or some other mutation. One of the cosmids that complemented Rm1681, pNtrA10, was chosen for further study and was found to contain the expected 3.5 kb BamHI/EcoRI fragment.

**Nucleotide Sequence of the R. meliloti ntrA Gene and Flanking DNA.**

To further characterize the R. meliloti ntrA gene, the DNA sequence of the 3.5 kb BamHI/EcoRI fragment that contains the gene was determined. The fragment was subcloned from the pLAFR1 cosmid pNtrA10 into pUC8 and the resultant plasmid pNtr3.5BE was used as the source of DNA for isolation of Bal31-deleted fragments for sequencing. The sequence data revealed three open reading frames (ORFs) (summarized in Fig. 1), including a central one of 1602 base pairs (see Table 3), the product of which has extensive homology to the K. pneumoniae ntrA gene product (27) (51.24 SD units by ALIGN analysis, Table 4) including 38% amino-acid identity.

**Table 3:** **Nucleotide Sequence of the 2.3 kb Region of the 3.5 kb BamHI/EcoRI Fragment Containing the R. meliloti ntrA gene and 5-Prime Region of ORF3**

```
1281  TCAGCCTCTGATGCCTGCCGGCGACGTGCATTCCGGCAGAACGGACGACCGCCGGCTGAA

      TCGGCACCTCCGCCGCCGTCCACGCTTGACCAAATTCCAGTAATAAGCAATTTTTGGGCC

1321  AACTAAGAAGATGGGCCCCGTCCAAGCGGACACGGCTCGGAGAACTCGACGGGAGTTTCAC
                                                              *****

      ACCAGCATGGCCCTTGTCCGCCAGCCTTCATCTGAGACAGTCGCAGTCGCTGGTCATGACG
      MetAlaLeuSerAlaSerLeuHisLeuArgGlnSerGlnSerLeuValMetThr

1441  CCGCAGCTGATGCAGTCGATCCAGCTGCTTCAGATGAACCATCTGGAGCTCAGCCATTTC
      ProGlnLeuMetGlnSerIleGlnLeuLeuGlnMetAsnHisLeuGluLeuSerHisPhe

      ATCGCGCAGGAAGTCGAGAAGAACCCGCTGCTGGAGGTCCAGCCGGCCGATGAGCCAACG
      IleAlaGlnGluValGluLysAsnProLeuLeuGluValGlnProAlaAspGluProThr

1561  ATATCGGATCGCGAGGATGCCGGTCCGCATCCGGCGGAGACCGGCGGCGAAACCGACGAG
      IleSerAspArgGluAspAlaGlyProHisProAlaGluThrGlyGlyGluThrAspGlu

      GCGGCCAGGTCAGAGCCGACCTTTACGACAGCGCCCATGTCGAGATCCGGCGAGAGCCTCAGC
      AlaAlaGlyGlnSerAspLeuTyrAspSerAlaMetSerArgSerGlyGluArgLeuSer

1681  GAAGGCCCTTGACGCCGACTTCGCCCAACGTCTTCCCCGACGATACGGCGCCGCAGCGGGCG
      GluGlyLeuAspAlaAspPheAlaAsnValPheProAspAspThrAlaProGlnArgAla
```

```
GATGCCCCCGAGCTTCTCGGCCAATGGAAATCCATGCCGGGTGCCGGCGACGCAGAGGGA
AspAlaProGluLeuLeuGlyGlnTrpLysSerMetProGlyAlaGlyAspAlaGluGly

1861 TACGATCTCGACGATTTCGTTGGCGGCCGCAAGACGTTGAGGGAGACGCTCGCCGAGCAG
     TyrAspLeuAspAspPheValGlyGlyArgLysThrLeuArgGluThrLeuAlaGluGln

     CTGCCCTTCGCACTCAGCGCTGTCTCCGATCGGCTGATCGCCCCGTATTTCATCGACCAG
     LeuProPheAlaLeuSerAlaValSerAspArgLeuIleAlaArgTyrPheIleAspGln

1921 CTCGATGATGCCGGCTACCTGCACGCCGACCTCGCCGAGACCGCCGAGACGCTGGGCGCG
     LeuAspAspAlaGlyTyrLeuHisAlaAspLeuAlaGluThrAlaGluThrLeuGlyAla

     GCAGGCGAGGACGTGGCCGCGCGTGCTGCACGTGCTTCAGCAGTTCGATCCGCCCGGCGTC
     AlaGlyGluAspValAlaArgValLeuHisValLeuGlnGlnPheAspProProGlyVal

2041 TTCGCGCGCACGCTTGGCGAATGTCTTGCCATTCAGCTGCGCGCCCGCAATCGTCTCGAC
     PheAlaArgThrLeuGlyGluCysLeuAlaIleGlnLeuArgAlaArgAsnArgLeuAsp

     CCGGCGATGGAAGCGCTCGTGGCCAATCTCGAGCTCCTGGCACGGCGCGATTTCGCAAGC
     ProAlaMetGluAlaLeuValAlaAsnLeuGluLeuLeuAlaArgArgAspPheAlaSer

2161 CTCAAGAAGATCTGCGGCGTCGACGAGGAAGACCTGATCGATATGCTCGCCGAGATCCGC
     LeuLysLysIleCysGlyValAspGluGluAspLeuIleAspMetLeuAlaGluIleArg

     AAGCTCGATCCGAAACCGGGCACCAGCTTCGAAACCGGTGTGTTCGAGGCCATCATTCCC
     LysLeuAspProLysProGlyThrSerPheGluThrGlyValPheGluAlaIleIlePro

2281 GACGTCGTCGTGCGCGCCGCACCCGATGGCGGCTGGCTTGTTGAGCTCAATCCGGATGCC
     AspValValValArgAlaAlaProAspGlyGlyTrpLeuValGluLeuAsnProAspAla

     CTGCCCCGCGGTTCTCGTCAACCACGACTATTTCACAGAAATATCGCGCTCCAGCCGGAAG
     LeuProArgValLeuValAsnHisAspTyrPheThrGluIleSerArgSerSerArgLys

2401 AACAGCGGCGAACAGGCCTTTCTCAACGAGTGCCTGCAGAATGCCAACTGGCTGACGCGC
     AsnSerGlyGluGlnAlaPheLeuAsnGluCysLeuGlnAsnAlaAsnTrpLeuThrArg

     AGCCTCGATCAGCGCGCCAGGACGATCATGAAGGTGGCGAGCGAAATCGTCCGGCAGCAG
     SerLeuAspGlnArgAlaArgThrIleMetLysValAlaSerGluIleValArgGlnGln

2521 GACGCCTTCCTCATCCACGGCGTCGGCCACCTGCGCCCCGCTGAACCTCAGGATCGTCGCG
     AspAlaPheLeuIleHisGlyValGlyHisLeuArgProLeuAsnLeuArgIleValAla

     GACGCGATCAAGATGCACGAGTCGACGGTGAGCCGGGTCACCTCCAACAAATACATGCTG
     AspAlaIleLysMetHisGluSerThrValSerArgValThrSerAsnLysTyrMetLeu

2641 ACCCCGCGCGGGCTCTTCGAATTGAAATATTTCTTCACGGTCTCGATCGGCTCGGCGGAA
     ThrProArgGlyLeuPheGluLeuLysTyrPhePheThrValSerIleGlySerAlaGlu

     AACGGCGATGCACATTCGGCCGAGTCCGTGCGCCATCGCATCCGCACGATGATCAATCAG
     AsnGlyAspAlaHisSerAlaGluSerValArgHisArgIleArgThrMetIleAsnGln
```

```
2781  GAAAGCGCCGATGCCGTGCTCTCGGACGACGACATCGTCGACGTCCTCAAGCAGGCCGGC
      GluSerAlaAspAlaValLeuSerAspAspAspIleValAspValLeuLysGlnAlaGly

      GTCGATATCGCCAGACGCACCGTCGCAAAATATCGCGAGGCGATGAGCATCCCCTCCTCC
      ValAspIleAlaArgArgThrValAlaLysTyrArgGluAlaMetSerIleProSerSer

2881  GTCCAGCGCCGCCGCGAGAAGCGGGCGCTGCCAAGGCCGCGGGATTCTGAGCGCTGCCGG
      ValGlnArgArgArgGluLysArgAlaLeuProArgProArgAspSerGluArgCysArg

      CAGGCCGCAAGCGCGTAAGCCGGGTTGCGCTTTGGGCTCTGCCCGGCGGAACCGCCGGGG
      GlnAlaAlaSerAlaEnd

3061  CCTGATGAACGTTTAATTCCACGACGCGCAATTGACTCTCCGCGGTACAGCGGATATGAG

      GCCGCCGCGCTTGCCTGAGATAGAACTCCGCGAGGCAGCGCGCGCTCACGATTTCGGTCG

3121  TTCAGGACTCCGGTCGCTGAAGTGCTTGGATGACGGGAGCGCTTGGAATAGACTGGGCAC

      GCAATTCACGAACAAGAGAGGAAACTCCATGAGTGTGCGTGTATCCGGTAAACATATGGA
                   *****       MetSerValArgValSerGlyLysHisMetGl

3241  AATCGGCGATTCGTTTCGCGTCCGCATCGGCGAGCAGATCGAGCAGGCCGTAACCAAATA
      uIleGlyAspSerPheArgValArgIleGlyGluGlnIleGluGlnAlaValThrLysTy

      TTTCGACGGAGGATATTCGAGCCAGGTCACTGTGGAAAAGTCAGGGTCGCGATTCAGCGC
      rPheAspGlyGlyTyrSerSerGlnValThrValGluLysSerGlySerArgPheSerAl

3361  CGACTGCAAGCTTCATCTCGATACGGGCGTGGTATTGCAGGCAAACGGCCAGGCGAACGA
      aAspCysLysLeuHisLeuAspThrGlyValValLeuGlnAlaAsnGlyGlnAlaAsnGl

      ACCACAGTCAGCCTTCGACGCGGCTTCGGAGCGCATCGAGAAGCGGCTCCGGCGCTACAA
      uProGlnSerAlaPheAspAlaAlaSerGluArgIleGluLysArgLeuArgArgTyrLy

3481  ACGCAAGCTCAAGGACCATCACAACGGCAACGGGCAGAATTC  3522
      sArgLysLeuLysAspHisHisAsnGlyAsnGlyGlnAsn
```

---

Nucleotides are numbered from the first base of the BamHI site (not shown). Possible ribosome binding sites are asterisked. The termination codon of ORF1 (see Figure 1) and an in-Frame ATG codon upstream of ORF3 are underlined. The position of the Tn5 insertion in strain Rm1681 (ntrA2::Tn5) is marked by the vertical arrow.

**Table 4: Optimum Alignment of ntrA Gene Products from R. meliloti (RMNTRA) and K. pneumoniae (KPNTRA)**

```
RMNTRA   1 MALSASLHLRQSQSLVMTPQLMQSIQLLQMNHLELSHFIAQEVEKNPLLEVQPAD
KPNTRA   1  MKQGLQLRLSQQLAMTPQLQQAIRLLQLSTLELQQELQQALDSNPLLE-QTDL
Common       L LR SQ L MTPQL Q I LLQ  LEL    Q   NPLLE Q

RMNTRA  56 EPTISDREDAGPHPAETGGETDEAAGQSDLYDSAMSRSGERLSEGLDADFANYFP
KPNTRA  53 HDEVETKEAEDRESLDTVDALEQKEMPEEL--------------PLDASYDEIYT
Common         E       T         L                     LDA

RMNTRA 111 DDTAPQRADAPELLGQYKSMPGAGDAEGYDLDDFY----GGRKTLRETLAEQLPF
KPNTRA  94 AGTPS-----------------GNGVDYQDDELPVYQGETTQSLQDYLMYQVEL
Common       T                   G   Y D          L   L Q

RMNTRA 162 ALSAVSDRLIARYFIDQLDDAGYLHADLAETAETLGAAG---EDVARVLHVLQQF
KPNTRA 131 TPFTDTDRAIATSIVDAVDDTGYLTISVEDIVESIGDDEIGLEEVEAVLKRIQRF
Common          DR IA   D  DD GYL      E  G      E V VL  Q F

RMNTRA 214 DPPGVFARTLGECLAIQL----RARNRLDPAMEALVANLELLARRDFASLKKICG
KPNTRA 186 DPVGVAAKDLRDCLLVQLSQFAKETPYIEEARLIISDHLDLLANHDFRSLMRVTR
Common     DP GV A  L CL QL           A        L LLA DF SL

RMNTRA 265 VDEEDLIDMLAEIRKLDPKPGTSFETGVFEAIIPDVYVRAAPDGGYLVELNPDAL
KPNTRA 241 LKEEVLKEAVNLIQSLDPRPGQSIQTGEPEYVIPDVLVRKVNDR-YVVELNSDSL
Common       EE L    I LDP PG S  TG E  IPDV VR   D  Y VELN D L

RMNTRA 320 PRVLVNHDYFTEISRSSRKNSGEQAFLNECLQNANYLTRSLDQRARTIMKVASEI
KPNTRA 295 PRLKINQQY-AAMGNSTRNDADGQ-FIRSNLQEARYLIKSLESRNDTLLRVSRCI
Common     PR  N Y    S R    QF   L Q A YL  SL R  T   V  I

RMNTRA 375 VRQQDAFLIHGVGHLRPLNLRIVADAIKMHESTVSRVTSNKYMLTPRGLFELKYF
KPNTRA 348 VEQQQAFFEQGEEFMKPMVLADIAQAVEMHESTISRVTTQKYLHSPRGIFELKYF
Common     V QQ AF  G     P L  A A  MHEST SRVT KY  PRG FELKYF

RMNTRA 430 FTYSIGSAENGDAHSAESVRHRIRTMINQESADAVLSDDDIVDVLKQAGVDIARR
KPNTRA 403 FSSHVNTEGGGEA-SSTAIRALYKKLIAAENPAKPLSDSKLTTMLSDQGIMVARR
Common     F      G A S   R      I E        LSD    L  G  ARR

RMNTRA 485 TVAKYREAMSIPSSVQRRREKRALPRPRDSERCRQAASA*
KPNTRA 457 TVAKYRESLSIPPSNQRKQLV*
Common     TVAKYRE SIP S QR
```

The start codon of the R. meliloti ntrA gene was provisionally assigned by comparison to the K. pneumoniae ntrA gene. The Tn5 insertions in Rm1680 (ntrA1::Tn5) position determined by restriction site mapping) and Rm1681 (ntrA2::Tn5, position determined by sequence analysis) mapped within the gene (Figure 1 and Table 3). The R. meliloti ntrA product is slightly larger than the K. pneumoniae ntrA product (524 versus 478 amino acids), but the products share homology throughout their length and have similar amino acid compositions.

The R. meliloti ntrA gene is preceded by an ORF that terminates 176 bp prior to the initiation codon of ntrA (Fig. 1, data not shown). Although the 176 bp region between ORF1 and ntrA (Table 3) does not contain strong stem-loop structures characteristic of the rho-independent transcriptional terminators, complementation data (see below) suggest that ORF1 is probably not part of an ntrA operon. Nevertheless, a similar ORF upstream of the K. pneumoniae ntrA gene was observed.

The R. meliloti 3.5. kb BamHI/EcoRI fragment also contained a third ORF (ORF3, Fig. 1) that initiated downstream of ntrA and continued through the EcoRI site. Two possible initiator ATG codons, one 98 bp and the other 251 bp downstream of the R. meliloti ntrA termination codon could be identified, but only the latter was preceded by a potential ribosome-binding site (GAGGA) (Table 3). Merrick and Gibbins (27) also identified a potential ORF that initiated 23 bp downstream of ntrA in K. pneumoniae, and the available 75 amino acids of this ORF share weak but possibly significant homology (3.95 SD units by ALIGN analysis) to the N-terminal 77 amino acids of ORF3, translated from the initiation codon 251 bp downstream of R. meliloti ntrA. Similarly to ORF1, complementation data (see below) indicated that ORF3 does not code for either an essential R. meliloti product or a product required for an NtrA[+] phenotype, assuming that Tn5 causes polar mutations in R. meliloti.

## Complementation Analysis of the ntrA Mutants.

To determine whether the R. meliloti ntrA gene is part of an operon containing ORF1, various DNA fragments that contained ntrA with or without ORF1 were tested for their ability to complement strain Rm1681 (ntrA2::Tn5). To isolate such a fragment, the nested deletions constructed for sequence determination were utilized. The replicative forms of M13 phage containing the deleted fragments were purified and the fragments excised with HindIII. (Each RF plasmid contained an HindIII site from the M13mp18 polylinker 5 prime of the fragment, and the HindIII site within the fragment 3 prime of the ntrA gene.) The HindIII fragments were then cloned in each orientation into pWB5A, a low copy number plasmid derived from pRK290 (Table 1), and pSUP106, a moderate copy number plasmid derived from RSF1010 (35), and the ability of the resultant plasmids to complement the succinate-negative and nitrate-negative phenotypes of strain Rm1681 determined.

The four fragments (F1 - F4) used are shown in Fig. 1; only the smallest fragment F4, which is deleted within the ntrA gene, was unable to complement strain Rm1681. The next smallest fragment F3 which contains none of the upstream gene and only 125 bp preceding the ntrA gened complemented strain Rm1681 to wild-type growth rates. To eliminate the possibility that the observed complementation was due to readthrough from vector promoters not previously described, the omega fragment which carries strong transcriptional stop signals at each end (13,34) was inserted as a BamHI fragment at the vector/insert junction upstream of ntrA in a pWB5A-based plasmid carrying F3, and in pSUP106-based plasmids carrying F3 in each orientation. The ability of the plasmids to complement strain Rm1681 was unaffected in each case.

## R. meliloti ntrA Is Constitutively Expressed.

The fragments in pSUP106 were cloned into the HindIII site within the tet gene, which is derived from pACYC184 (35). Insertion of a DNA fragment in this site disrupts the promoter of the tet gene but leaves the coding sequence intact (25). Therefore a promoter within the inserted fragment that reads through into the tet gene can cause expression of tetracycline resistance. E. coli MM294 derivatives containing pSUP106 with F1, F2, F3 or F4 in either orientation were found to be tetracycline-sensitive; however, R. meliloti Rm1681 derivatives containing pSUP106 with F1, F2 or F3 in the orientation such that ntrA was transcribed in the same direction as tet were tetracycline-resistant, even when cultured on LB medium. The pSUP106 derivative containing the omega fragment upstream of F3 also conferred tetracycline resistance. Examination of the nucleotide sequence of the tet gene (25) indicates that ORF3 is terminated immediately after the HindIII site in these constructions (sequence in frame AAG CTT TAA). R. meliloti strains containing pSUP106 with F4 in either orientation or F1, F2 or F3 in the orientation such that ntrA was transcribed in the opposite direction to tet were tetracycline-sensitive. These results suggest that the R. meliloti ntrA promoter is not expressed in E. coli, but is constitutively expressed in R. meliloti and reads through ORF3.

## LITERATURE CITED

1. Bankier, A.T., et al. In R.A. Flavell (ed.), Techniques in Life Sciences, B$_5$: Nucleic Acid Biochemistry. Elsevier/North Holland, Limerick, Ireland p. 1-34 (1983).

2. Better M., et al. Cell 35:479-485 (1983).

3. Bueno, R., J. Bacteriol. 164:816-822 (1985).

4. Buikema, W.B., et al., Nucleic Acids Res. 13:4539-4555 (1985).

5. Castano, I., et al., Molec. Gen. Genet. 195:228-233 (1984).

6. Dayhoff, M.O., et al., Methods in Enzymology 91:524-545 (1983).

7. de Bruijn, F.J., et al., Molec. Gen. Genet. 192:342-353 (1983).

8. Devereux, J., et al., Nucleic Acids Res. 12:387-395 (1984).

9. Ditta, G., et al., Proc. Natl. Acad. Sci. (USA) 77:7347-7351 (1980).

10. Dixon, R., Mol. Gen. Genet. 203:129-136 (1986).

11. Drummond, M., et al., EMBO J. 5:441-447 (1986).

12. Feinberg, A.P., et al., Biochem. 132:6-13 (1983).

13. Frey, J., Gene 36:143-150 (1985).

14. Friedman, A.M., et al., Gene 18:289-296 (1982).

15. Garcia, E., et al., Proc. Natl. Acad. Sci. (USA) 74:1662-1666 (1977).

16. Gussin, G.N., et al., Ann. Rev. Genet. 20:567-591 (1986).

17. Hirschmann, J., et al., Proc. Natl. Acad. Sci (USA) 82:7525-7529 (1985).

18. Holmes, D.S., et al., Anal. Biochem. 114:193-197 (1981).

19. Hunt, T.P., et al., Proc. Natl. Acad. Sci. (USA) 82:8453-8457 (1985).

20. Inouye, S., et al., Proc. Natl. Acad. Sci. (USA) 81:1688-1691 (1984).

21. Johnson, K., et al., J. Biol. chem. 261:15703-15708 (1986).

22. Kustu, S., et al., Regulation of Gene Expression: Symposium of the Society for General Microbiology, I. Booth and C. Higgins (eds.), pp. 139-154, Cambridge University Press, Cambridge.

23. MacNeil, T., et al., Mol. Gen. Genet. 188:325-333 (1982).

24. Magasanik, B., Ann. Rev. Genet. 16:135-168 (1982).

25. Maniatis, T., et al., Molecular Cloning - A Laboratory Manual. Cold Spring Harbor Laboratory, Cold Spring Harbor, New York (1982).

26. Meade, H.M., et al., J. Bacteriol. 149:114-122 (1982).

27. Merrick, M.J., et al., Nucleic Acids Res. 13:7607-7620 (1985).

28. Merrick, M.J., et al.,, Gene 35:297-303 (1985).

29. Miller, J.H., Experiments in Molecular Genetics, p. 352-355. Cold Spring Harbor Laboratory, Cold Spring Harbor, New York (1972).

30. Minton, N.P., et al., J. Mol. Appl. Genet. 3:26-35 (1985).

31. Ninfa, A.J., et al.,, Proc. Natl. Acad. Sci. (USA) 83:5909-5913 (1986).

32. Nixon, B.T., et al., Proc. Natl. Acad. Sci. (USA) 83:7850-7854 (1986).

33. Poncz, M., et al., Proc. Natl. Acad. Sci. (USA) 79:4298-4302 (1982).

34. Prentki, P., et al., Gene 29:303-313 (1984).

35. Priefer, U.B., et al.,, J. Bacteriol. 163:324-330 (1985).

36. Ronson, C.W., et al.,, Genes involved in the carbon metabolism of bacteriods, p. 201-207. In H.J. Evans, P.J. Bottomley and W.E. Newton (eds.), Nitrogen Fixation Research Progress. Martinus Nijhoff, Dordrecht (1985).

37. Ronson, C.W., et al., J. Bacteriol. 160:903-909 (1984).

38. Ronson, C.W., et al., Proc. Natl. Acad. Sci. (USA) 78:4284-4288 (1981).

39. Ronson, C.W. et al., J. Gen. Microbiol. 112:77-88 (1979).

40. Schofield, P.R., et al., Nucleic Acids Res. 14:2891-2903 (1986).

41. Scott, D.B., et al., Arch. Microbiol. 139:151-157 (1984).

42. Staden, R., Nucleic Acids Res. 10:4731-4751 (1982).

43. Sundaresan, V., et al., Nature 301:728-732 (1983).

44. Szeto, W.W., et al., J. Bacteriol., in press (JB#623-86).

45. Szeto, W.W., et al., Cell 36:1035-1043 (1984).

46. Vieira, J., et al., Gene 19:259-268 (1982).

47. Yanisch-Perron, C., et al., Gene 33:103-119 (1985).

**Claims**

1. A cloned Rhizobium meliloti ntrA gene comprising the deoxyribonucleotide sequence depicted in Table 3.

2. A plasmid, comprising the cloned ntrA gene of Claim 1.

3. The plasmid of claim 2, wherein said plasmid is pNtrA10.

4. A bacterial host transformed with the plasmid of claim 2.

5. The host of claim 5, wherein said host is E. coli.

6. NtrA having the amino acid sequence depicted in Table 3 .

7. A method of obtaining a bacterial cell having a mutation in the ntrA gene, which comprises

a) transforming a culture of bacterial cells with a plasmid having an ntrA regulatable gene fusion, said gene fusion resulting from the linkage of an ntrA regulated promoter with a gene whose product has detectable activity,

b) screening said transformed cells for said bacterial cell having a mutation in the ntrA gene, said cell being identified by the absence of said detectable activity, and

c) separating said bacterial cell from the other cells of said culture.

8. The method of claim 7, wherein said regulatable gene fusion is the lacZ fusion.

9. The method of claim 7, wherein said ntrA regulated promoter is the dctA promoter.

Claims for the following Contracting State : ES

1. A method of preparing the gene product NtrA of the Rhizobium meliloti ntrA (rpoN) gene having the amino acid sequence depicted in Table 3, which comprises expressing in a heterologous host cell the DNA sequence depicted in Table 3.

2. The method of claim 1, wherein the ntrA gene is cloned into a plasmid and the host cell is transformed with said plasmid.

3. The method of claim 2, wherein the plasmid is pNtrA10.

4. The method of one or more of the preceding claims, wherein the host cell is a bacterium.

5. The method of claim 4, wherein the host is E. coli.

6. A method of obtaining a bacterial cell having a mutation in the ntrA gene, which comprises

a) transforming a culture of bacterial cells with a plasmid having an ntrA regulatable gene fusion, said gene fusion resulting from the linkage of an ntrA regulated promoter with a gene whose product has detectable activity,

b) screening said transformed cells for said bacterial cell having a mutation in the ntrA gene, said cell being identified by the absence of said detectable activity, and

c) separating said bacterial cell from the other cells of said culture.

7. The method of claim 6, wherein said regulatable gene fusion is the lacZ fusion.

8. The method of claim 6, wherein said ntrA regulated promoter is the dctA promoter.

Figure 1